(19) Europäisches Patentamt
European Patent Office
Office européen des brevets

(11) **EP 3 739 592 A1**

(12) **EUROPÄISCHE PATENTANMELDUNG**

(43) Veröffentlichungstag:
**18.11.2020 Patentblatt 2020/47**

(51) Int Cl.:
***G16H 30/40*** *(2018.01)*

(21) Anmeldenummer: **19174633.8**

(22) Anmeldetag: **15.05.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH**
**91052 Erlangen (DE)**

(72) Erfinder: **Lauer, Lars**
**91077 Neunkirchen (DE)**

(54) **DEZENTRALISIERT GESTEUERTE BILDGEBUNGSBASIERTE PATIENTENDATENGEWINNUNG**

(57) Es wird ein dezentralisiert gesteuertes Bildgebungsverfahren (100, 300, 400, 500) beschrieben. Bei dem Bildgebungsverfahren wird eine über ein Datenübertragungsnetz mit einer Kommunikationseinrichtung (21k) einer medizintechnischen bildgebenden Anlage (21) verbindbare Steuerungsinstanz (24) ermittelt und ausgewählt. Es wird eine netzbasierte Kommunikationsverbindung zwischen der Kommunikationseinrichtung (21k) der medizintechnischen bildgebenden Anlage (21) und der Steuerungsinstanz (24) initialisiert und es wird ein Bildaufnahmevorgang von einem Untersuchungsbereich (FoV) eines Patienten (P) durch die Steuerungsinstanz (24) über die netzbasierte Kommunikationsverbindung ferngesteuert. Es wird auch ein Befundungsdatengewinnungsverfahren beschrieben. Es wird ferner ein dezentralisiertes Bilddatengewinnungssystem (20, 130) beschrieben. Weiterhin wird ein Befundungsdatengewinnungssystem beschrieben. Überdies wird ein Verfahren zum Bereitstellen eines trainierten automatisierten Steuerungssystems beschrieben. Es wird ferner ein Trainingssystem (120) beschrieben. Außerdem wird ein Trainingsdatengewinnungssystem (130) beschrieben.

FIG 1

Printed by Jouve, 75001 PARIS (FR)

EP 3 739 592 A1

**Beschreibung**

Die Erfindung betrifft ein dezentralisiert gesteuertes Bildgebungsverfahren. Weiterhin betrifft die Erfindung ein Befundungsdatengewinnungsverfahren. Überdies betrifft die Erfindung ein dezentralisiertes Bilddatengewinnungssystem. Ferner betrifft die Erfindung ein Befundungsdatengewinnungssystem. Außerdem betrifft die Erfindung ein Verfahren zur Gewinnung von Trainingsdaten. Darüber hinaus betrifft die Erfindung ein Verfahren zum Bereitstellen eines trainierten automatisierten Steuerungssystems. Daneben betrifft die Erfindung ein Trainingsdatengewinnungsystem. Zudem betrifft die Erfindung ein Trainingssystem.

Medizintechnische bildgebende Systeme sind für die Diagnosestellung in vielen Fällen unerlässlich. Allerdings wird für eine effektive Nutzung solcher Geräte eine Vielzahl an qualifizierten Personen benötigt, die insbesondere in weniger entwickelten Ländern nicht immer verfügbar sind. Beispielsweise wird für die Vorbereitung eines Bildgebungsvorgangs, insbesondere für die Festlegung eines Bildgebungsprotokolls, qualifiziertes Personal benötigt. Für diese Aufgabe steht herkömmlich eine medizinischtechnische Assistentin (MTA) zur Verfügung. Auch für eine Auswertung der Bilddaten wird Fachpersonal, d.h. ein qualifizierter Radiologe benötigt. Ist Fachpersonal knapp, so wird herkömmlich die Steuerung und Auswertung der Bildgebung zentralisiert. Zwischen dem Bildgebungssystem und einem virtuellen qualifizierten Bediener besteht eine Kommunikationsverbindung, über die der Bildgebungsvorgang überwacht und gesteuert werden kann. Allerdings muss auch bei einer solchen Lösung immer ausreichend qualifiziertes Personal in der zentralen Einrichtung vorgehalten werden. Ein ähnliches Problem besteht auch bei der Auswertung der erzeugten Bilddaten. Beispielsweise wäre es wünschenswert, auf der Basis von Bilddaten auch während des Nachtbetriebs eines Krankenhauses zeitnah eine Diagnose stellen zu können. Meist sind lokal zur Nachtzeit aber keine Radiologen für die Befundung der Bilddaten verfügbar.

Es besteht also das Problem, medizintechnische Bildgebungsverfahren und Auswertungen der erzeugten Bilddaten auch unabhängig von lokal vorhandenem geeignetem Fachpersonal durchführen zu können.

Diese Aufgabe wird durch ein dezentralisiert gesteuertes Bildgebungsverfahren gemäß Patentanspruch 1, ein Befundungsdatengewinnungsverfahren gemäß Patentanspruch 4, ein dezentralisiertes Bilddatengewinnungssystem gemäß Patentanspruch 8, ein Befundungsdatengewinnungssystem gemäß Patentanspruch 10, ein Verfahren zur Gewinnung von Trainingsdaten gemäß Patentanspruch 11, ein Verfahren zum Bereitstellen eines trainierten automatisierten Steuerungssystems gemäß Patentanspruch 13, ein Trainingsdatengewinnungssystem gemäß Patentanspruch 15 und ein Trainingssystem gemäß Patentanspruch 16 gelöst.

Bei dem erfindungsgemäßen dezentralisiert gesteuerten Bildgebungsverfahren wird eine Steuerungsinstanz, die über ein Datenübertragungsnetz mit einer Kommunikationseinrichtung einer medizintechnischen bildgebenden Anlage verbindbar ist, ermittelt und ausgewählt. Als Datenübertragungsnetz kann ein Verbund von Rechnernetzwerken, wie zum Beispiel das Internet, verwendet werden. Als medizintechnische bildgebende Anlage kann zum Beispiel ein CT-Gerät oder eine MR-Anlage genutzt werden. Unter einer Steuerungsinstanz wird eine Einrichtung verstanden, welche für den Betrieb einer medizintechnischen bildgebenden Anlage notwendige Steuerungsinformationen bzw. Steuerungsbefehle bereitstellen kann. Diese kann eine Kommunikationsschnittstelle für eine medizinischtechnische Assistentin umfassen oder eine automatisierte Rechnereinheit, welche einen Algorithmus bzw. ein dem Algorithmus folgendes Programm zur Steuerung eines Bildgebungsvorgangs aufweist. Der Algorithmus kann modellbasiert oder auf künstlicher Intelligenz basiert erzeugt werden. Zwischen der Kommunikationseinrichtung der medizintechnischen bildgebenden Anlage und der Steuerungsinstanz wird eine netzbasierte Kommunikationsverbindung initialisiert. Durch die Steuerungsinstanz wird über die netzbasierte Kommunikationsverbindung ein Bildaufnahmevorgang von einem Untersuchungsbereich eines Patienten ferngesteuert. D.h., die Steuerungsinstanz und die medizintechnische bildgebende Einrichtung können sich an voneinander weit entfernten Orten befinden. Die beiden Einheiten werden für den Bildgebungsvorgang miteinander über das Datenübertragungsnetz verbunden. Vorteilhaft kann die Steuerungsinstanz flexibel für die Fernsteuerung von an unterschiedlichen Orten befindlichen medizintechnischen Einrichtungen genutzt werden. Umgekehrt kann sich ein Benutzer einer medizintechnischen bildgebenden Einrichtung über das Datenübertragungsnetz eine aktuell verfügbare Steuerungsinstanz suchen, wodurch eine hohe Flexibilität, Effizienz und zeitnahe Verfügbarkeit eines kompetenten Betriebs einer medizintechnischen bildgebenden Anlage ermöglicht wird, auch dann, wenn zum Beispiel vor Ort kein Fachpersonal zur Verfügung steht, wie es zum Beispiel währen der Nachtzeit der Fall ist. Es ist beispielsweise ein Betrieb nur mit Krankenschwestern und durch einen während der Nachtzeit in einer anderen Zeitzone verfügbaren medizinischtechnischen Assistenten oder mit Hilfe von geeigneten Steuerungsprogrammen, die zum Beispiel cloudbasiert realisiert sind, möglich.

Bei dem erfindungsgemäßen Befundungsdatengewinnungsverfahren wird das erfindungsgemäße dezentralisiert gesteuerte Bildgebungsverfahren durchgeführt. Weiterhin wird netzbasiert ein mit der Kommunikationseinrichtung einer medizintechnischen bildgebenden Anlage verbindbarer Expertenarbeitsplatz, vorzugsweise eine Teleradiologenstelle, aus einer Mehrzahl von Expertenarbeitsplätzen ermittelt und ausgewählt. Der Expertenarbeitsplatz kann optional einen Expertenarbeitsplatz für eine qualifizierte Bedienperson, einen sogenannten Teleradiologen, aufweisen. Die bei dem Bildaufnahmevorgang erzeugten Bilddaten werden an den ausgewählten Expertenarbeitsplatz übermittelt und es werden Befundungsdaten von dem Expertenarbeitsplatz ausgegeben. Die Befundungsdaten können optional von dem Expertenarbeitsplatz an die Kommunikationseinrichtung der medizintechnischen bildgebenden Anlage und an eine Benutzerperson oder einen Patienten, alternativ an den Patienten nach Hause, geschickt werden. Der Expertenarbeitsplatz kann analog zu der Steuerungsinstanz auch automatisiert werden, d.h. die Bilddaten können von einem im Datenübertragungsnetz gespeicherten Computerprogramm bearbeitet werden.

Das erfindungsgemäße dezentralisierte Bilddatengewinnungssystem weist eine medizintechnische bildgebende Anlage auf. Teil des erfindungsgemäßen dezentralisierten Bilddatengewinnungssystems ist auch eine Vermittlungseinrichtung zum Ermitteln und Auswählen einer über ein Datenübertragungsnetz mit einer Kommunikationseinrichtung der medizintechnischen bildgebenden Anlage verbindbaren Steuerungsinstanz. Die Vermittlungseinrichtung kann sowohl hardwarebasiert als auch softwarebasiert ausgebildet sein. Zum Beispiel kann sie ein Vermittlungsprogramm umfassen, welches eine medizintechnische bildgebende Einrichtung an eine Steuerungsinstanz vermittelt. Das erfindungsgemäße dezentralisierte Bilddatengewinnungssystem umfasst überdies eine Initialisierungseinrichtung zum Initialisieren einer netzbasierten Kommunikationsverbindung zwischen der Kommunikationseinrichtung der medizintechnischen bildgebenden Anlage und der Steuerungsinstanz. Weiterhin weist das erfindungsgemäße dezentralisierte Bilddatengewinnungssystem eine Steuerungsinstanz zum Fernsteuern eines Bildaufnahmevorgangs von einem Untersuchungsbereich eines Patienten über die netzbasierte Kommunikationsverbindung auf. Die Steuerungsinstanz kann sich weit entfernt von der medizintechnischen bildgebenden Anlage befinden. Das erfindungsgemäße dezentralisierte Bilddatengewinnungssystem teilt die Vorteile des erfindungsgemäßen dezentralisiert gesteuerten Bildgebungsverfahrens.

Das erfindungsgemäße Befundungsdatengewinnungssystem weist das erfindungsgemäße dezentralisierte Bilddatengewinnungssystem auf. Zudem umfasst das erfindungsgemäße Befundungsdatengewinnungssystem eine Vermittlungseinrichtung zum netzbasierten Ermitteln und Auswählen eines mit der Kommunikationseinrichtung einer medizintechnischen bildgebenden Anlage verbindbaren Expertenarbeitsplatzes aus einer Mehrzahl von Expertenarbeitsplätzen. Der Expertenarbeitsplatz weist optional einen Expertenarbeitsplatz für eine qualifizierte Bedienperson auf. Teil des erfindungsgemäßen Befundungsdatengewinnungssystems ist auch eine Initialisierungseinrichtung zum Initialisieren einer netzbasierten Kommunikationsverbindung zwischen der Kommunikationseinrichtung der medizintechnischen bildgebenden Anlage und dem ausgewählten Expertenarbeitsplatz zum Übermitteln der bei dem Bildaufnahmevorgang erzeugten Bilddaten an den ausgewählten Expertenarbeitsplatz. Das erfindungsgemäße Befundungsdatengewinnungssystem umfasst auch eine Ausgangsschnittstelle zum Ausgeben von Befundungsdaten von dem Expertenarbeitsplatz. Das erfindungsgemäße Befundungsdatengewinnungssystem teilt die Vorteile des erfindungsgemäßen Befundungsdatengewinnungsverfahrens.

Bei dem erfindungsgemäßen Verfahren zur Gewinnung von Trainingsdaten, welches vorzugsweise unter Anwendung des erfindungsgemäßen dezentralisiert gesteuerten Bildgebungsverfahrens erfolgt, wird ein automatisiertes Bilddatengewinnungssystem mit einer medizintechnischen bildgebenden Anlage, einer Steuerungsinstanz, einer Kommunikationseinrichtung und einem automatisierten Steuerungssystem eingerichtet. Das automatisierte Steuerungssystem dient dazu, einen Bildgebungsprozess der medizintechnischen bildgebenden Anlage zu steuern. Bei dem automatisierten Bilddatengewinnungssystem handelt es sich vorzugsweise um das erfindungsgemäße dezentralisierte Bilddatengewinnungssystem. Bei dem erfindungsgemäßen Verfahren zur Gewinnung von Trainingsdaten wird zur Gewinnung von Trainingsdaten das automatisierte Steuerungssystem temporär durch einen Expertenarbeitsplatz mit einem Experten ersetzt. D.h., zur Gewinnung von Trainingsdaten wird der automatisierte Betrieb durch einen Betrieb mit einem Experten ersetzt. Weiterhin wird im Rahmen des Verfahrens eine Kommunikation zwischen dem Experten und einer Benutzerperson und/oder der bildgebenden medizintechnischen Anlage, die im Rahmen der Durchführung eines gesteuerten Bildgebungsverfahrens erfolgt, protokolliert. Die Kommunikation kann Sprache, Übermittlung von Bilddaten, Steuerungsdaten, Messdaten etc. umfassen. Optional werden die dabei protokollierten Daten aufbereitet. Bei der Aufbereitung der Daten kann zum Beispiel eine Transformation in ein verarbeitbares Format erfolgen. Außerdem werden die protokollierten Daten und/oder aufbereiteten protokollierten Daten als Trainingsdaten bereitgestellt. Trainingsdaten umfassen sogenannte Eingangstrainingsdaten und Ausgangstrainingsdaten. Sie dienen zum Trainieren eines neuronalen Netzes, das an die gesammelten Trainingsdaten angepasst wird. Im Rahmen des Trainings findet ein rekursiver Anpassungsprozess an die vorhandenen Trainingsdaten statt. Vorteilhaft können die ohnehin bei einer durch Experten gesteuerten Bildgebung anfallenden Eingangs- und Ausgangsdaten für eine weitergehende Flexibilisierung und Automatisierung der Bildgebung genutzt werden. Insbesondere häufig benötigte Bildgebungsprozesse bzw. Anwendungen auf bestimmte Körperteile, liefern entsprechend reichhaltiges Datenmaterial, so dass diese Anwendungen als erstes bzw. bevorzugt mit Hilfe von Trainingsprozessen und neuronalen Netzen automatisiert werden können.

Bei dem erfindungsgemäßen Verfahren zum Bereitstellen eines trainierten automatisierten Steuerungssystems werden gelabelte Eingangstrainingsdaten empfangen. Weiterhin werden gelabelte Ausgangstrainingsdaten empfangen, wobei die Ausgangstrainingsdaten mit den Eingangstrainingsdaten zusammenhängen. Als gelabelte Trainingsdaten sollen Daten verstanden werden, die miteinander in funktionellem Bezug stehen und einander zugeordnet sind. Bei dem erfindungsgemäßen Verfahren zum Bereitstellen eines trainierten automatisierten Steuerungssystems wird eine Funktion basierend auf den Eingangstrainingsdaten und den Ausgangstrainingsdaten mit Hilfe eines künstlichen neuronalen Netzes trainiert und es wird die trainierte Funktion für ein automatisiertes Steuerungssystem bereitgestellt. Das automatisierte Steuerungssystem dient vorzugsweise der Bildgebungssteuerung. Vorteilhaft kann die Steuerung von Bildgebungsprozessen automatisiert werden und so unabhängig von der Verfügbarkeit von Experten gemacht werden.

Das erfindungsgemäße Trainingsdatengewinnungssystem weist ein automatisiertes Bilddatengewinnungssystem auf. Das automatisierte Bilddatengewinnungssystem weist eine medizintechnische bildgebende Anlage, eine Steuerungsinstanz, eine Kommunikationseinrichtung und ein automatisiertes Steuerungssystem auf. Das automatisierte Bilddatengewinnungssystem umfasst vorzugsweise das erfindungsgemäße dezentralisierte Bilddatengewinnungssystem. Das erfindungsgemäße Trainingsdatengewinnungssystem umfasst außerdem einen Expertenarbeitsplatz, welcher zur Gewinnung von Trainingsdaten das automatisierte Steuerungssystem temporär ersetzt. Teil des erfindungsgemäßen Trainingsdatengewinnungssystems ist auch eine Protokolleinheit zum Protokollieren der Kommunikation zwischen dem Experten und einer Benutzerperson und/oder der bildgebenden medizintechnischen Anlage. Optional umfasst das erfindungsgemäße Trainingsdatengewinnungssystem auch eine Datenaufbereitungseinheit zum Aufbereiten der protokollierten Daten. Daneben weist das erfindungsgemäße Trainingsdatengewinnungssystem eine Ausgangsschnittstelle zum Ausgeben der protokollierten Daten und/oder aufbereiteten Daten als Trainingsdaten auf. Das erfindungsgemäße Trainingsdatengewinnungssystem teilt die Vorteile des erfindungsgemäßen Verfahrens zur Gewinnung von Trainingsdaten.

Das erfindungsgemäße Trainingssystem weist eine erste Trainingsschnittstelle zum Empfangen von Eingangstrainingsdaten auf. Teil des erfindungsgemäßen Trainingssystems ist auch eine zweite Trainingsschnittstelle zum Empfangen von Ausgangstrainingsdaten, wobei die Ausgangstrainingsdaten mit den Eingangstrainingsdaten zusammenhängen. Das erfindungsgemäße Trainingssystem umfasst zudem eine Trainingsrechnereinheit zum Trainieren einer Funktion zur automatisierten Steuerung basierend auf den Eingangstrainingsdaten und den Ausgangstrainingsdaten. Teil des erfindungsgemäßen Trainingssystems ist auch eine dritte Trainingsschnittstelle zum Bereitstellen der Trainingsfunktion für ein automatisiertes Steuerungssystem. Das erfindungsgemäße Trainingssystem teilt die Vorteile des erfindungsgemäßen Verfahrens zum Bereitstellen eines trainierten automatisierten Steuerungssystems.

Die wesentlichen Komponenten der erfindungsgemäßen Systeme können zum überwiegenden Teil in Form von Softwarekomponenten ausgebildet sein. Dies betrifft insbesondere die Vermittlungseinrichtung, die Initialisierungseinrichtung und die Steuerungsinstanz des erfindungsgemäßen dezentralisierten Bilddatengewinnungssystems, die Vermittlungseinrichtung, die Initialisierungseinrichtung des erfindungsgemäßen Befundungsdatengewinnungssystems, die Trainingsrechnereinheit des Trainingssystems sowie die Protolleinheit und die Datenaufbereitungseinheit des Trainingsdatengewinnungssystems. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher verwendete Datenübertragungsnetze auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung eines oder mehrerer Teilsysteme eines solchen Datenübertragungsnetzes ladbar ist, mit Programmabschnitten, um die durch Software realisierbaren Schritte der erfindungsgemäßen Verfahren auszuführen, wenn das Programm in dem Datenübertragungsnetz ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z.B. Hardware-Schlüssel (Dongles etc.) zur Nutzung der Software, umfassen.

Zum Transport zu dem Teilsystem und/oder zur Speicherung an oder in diesem Teilsystem kann ein computerlesbares Medium, beispielsweise ein Memorystick, eine Festplatte oder ein sonstiger transportabler oder fest eingebauter Datenträger dienen, auf welchem die von einer Rechnereinheit der Steuereinrichtung einlesbaren und ausführbaren Programmabschnitte des Computerprogramms gespeichert sind. Die Rechnereinheit kann z. B. hierzu einen oder mehrere zusammenarbeitende Mikroprozessoren oder dergleichen aufweisen. Die Rechnereinheit kann zum Beispiel Teil eines Terminals oder einer Steuereinrichtung eines bildgebenden Systems, wie zum Beispiel eine MR-Anlage oder eine CT-Anlage, sein, sie kann aber auch Teil eines entfernt positionierten Serversystems innerhalb des Datenübertragungsnetzes sein.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung die verschiedenen Merkmale unterschiedlicher Ausführungsbeispiele und Ansprüche auch zu neuen Ausführungsbeispielen kombiniert werden.

In einer Ausgestaltung des erfindungsgemäßen dezentralisiert gesteuerten Bildgebungsverfahrens weist die Steuerungsinstanz einen Expertenarbeitsplatz auf, an welchem Steuerungsdaten erfasst und über die netzbasierte Kommunikationsverbindung zur Protokollerstellung und zur Durchführung des Bildgebungsverfahrens übermittelt werden. Vorteilhaft kann ein von einer qualifizierten Person besetzter Expertenarbeitsplatz einem beliebig weit entfernten Bildgebungssystem zugeordnet werden, so dass Humankapazitäten flexibler und effektiver genutzt werden können.

In einer Ausgestaltung des erfindungsgemäßen dezentralisiert gesteuerten Bildgebungsverfahrens erfolgt das Fernsteuern des Bildaufnahmevorgangs auf Basis eines über die netzbasierte Kommunikationsverbindung geführten Dialogs zwischen einer qualifizierten Bedienperson an dem Expertenarbeitsplatz und einer am Ort der medizintechnischen bildgebenden Anlage befindlichen Benutzerperson. Bei dieser Variante erhält der Benutzer Anweisungen zur Bedienung der von ihm genutzten bildgebenden Einrichtung von der qualifizierten Bedienperson. Vorteilhaft kann der Benutzer auf Basis der Anweisungen selbst die Kommunikation zur Vorbereitung der Bildgebung führen. Eine direkte Ansteuerung des bildgebenden Systems von dem Expertenarbeitsplatz wird nicht benötigt.

Bevorzugt kann zumindest eine der qualifizierten Bedienpersonen durch ein auf künstlicher Intelligenz basierendes, mit Hilfe von Trainingsdaten trainiertes automatisiertes Steuerungssystem simuliert werden. Bei dieser vorteilhaften Variante kann die qualifizierte Bedienperson durch einen Algorithmus bzw. ein einen Algorithmus umsetzendes Computerprogramm ersetzt werden, so dass eine erhöhte Einsatzbereitschaft, geringere Kosten und eine verbesserte Verfügbarkeit der medizintechnischen Dienstleistungen erreicht werden kann.

Ein Einsatz des trainierten automatisierten Steuerungssystems kann in Abhängigkeit davon gestartet werden, ob eine ausreichende Anzahl von Trainingsdatensätzen für den Trainingsprozess des jeweiligen automatisierten Steuerungssystems zur Verfügung stehen. Vorteilhaft wird die Automatisierung der Steuerung der Bildgebung oder der Befundung auf Anwendungen konzentriert, die am häufigsten gebraucht werden und zudem aufgrund der breitesten Datengrundlage auch am zuverlässigsten automatisierbar sind.

Bevorzugt wird eine Klassifikation von Aufträgen zur Bildgebung oder Befundung in Aufträge, welche für eine virtuelle qualifizierte Bedienperson oder ein automatisiertes System geeignet sind, auf künstlicher Intelligenz basierend durchgeführt. D.h., sogar die Entscheidung selbst, ob die Fernsteuerung der Bildgebung oder der Befundung durch eine Person oder automatisiert erfolgend soll, wird durch ein trainiertes KIbasiertes Computerprogramm getroffen (KI = Abkürzung für künstliche Intelligenz). Vorteilhaft kann auch dieser Entscheidungsprozess unabhängig von vorhandenem qualifiziertem Personal realisiert werden und es können auf diese Weise Personalkosten gespart werden und die Verfügbarkeit dieses Entscheidungsprozesses erhöht werden. Die Nutzung von KIbasierten Lösungen kann zum Beispiel von der jeweiligen länderspezifischen Gesetzgebung abhängig gemacht werden. Beispielsweise wird für eine solche Anwendung eine offizielle Freigabe einer bestimmten Untersuchung für eine automatisierte Steuerung benötigt. Liegt diese Freigabe in einem bestimmten Staat vor, so kann die jeweilige Untersuchung in diesem Staat basiert durchgeführt werden, während dieselbe Untersuchung eines Patienten, der sich in einem Staat ohne Freigabe befindet, zum Beispiel mit Hilfe einer virtuellen Bedienperson erfolgt.

Die medizintechnische bildgebende Anlage des erfindungsgemäßen dezentralisierten Bilddatengewinnungssystems umfasst bevorzugt eine Anlage der folgenden Anlagentypen:

- eine CT-Anlage,
- eine MR-Anlage.

Die genannten Typen von bildgebenden Anlagen benötigen umfangreiche Kenntnisse bei der Bedienung und Steuerung der bildgebenden Prozesse. Es ist gerade bei dem Betrieb dieser Art von Systemen besonders vorteilhaft, wenn ein Betrieb unabhängig von lokal vorhandenem geeignetem Personal ermöglicht werden kann.

In einer bevorzugten Variante des erfindungsgemäßen Verfahrens zur Gewinnung von Trainingsdaten umfassen die Trainingsdaten als Eingangstrainingsdaten mindestens eine der folgenden Datenarten:

- Patientendaten,
- aufgabenspezifische Daten, vorzugsweise Untersuchungsdaten,
- die medizintechnische bildgebende Anlage betreffende technische Daten,
- Scoutbilddaten,
- Bilddaten.

Die genannten Eingangstrainingsdaten umfassen Informationen, die als Grundlage für die Durchführung der jeweiligen Teilprozesse eines Bildgebungsprozesses oder Befundungsprozesses benötigt werden.

Weitere Datenquellen für Eingangstrainingsdaten sind zum Beispiel:

- die Patienten-Anamnese des zuweisenden Arztes sowie Daten zu Vorbefunden aus der Patientenakte,
- Daten einer Patienten-Überwachungskamera in der medizintechnischen bildgebenden Anlage oder von einer Deckenkamera im Untersuchungsraum. Diese Daten können zur Plausibilisierung von anderen Daten, wie zum Beispiel dem Patientengewicht, der Größe und dem Geschlecht, genutzt werden. Weiterhin können die genannten Kameras im Fall der Nutzung eines MR-Systems zur Untersuchung zur Überwachung der korrekten Positionierung von Empfangsspulen durch den Bediener vor Ort eingesetzt werden.

Als Ausgangstrainingsdaten umfassen die Trainingsdaten mindestens eine der folgenden Datenarten:

- Steuerungsdaten, die durch Experten, beispielsweise Bedienpersonen und/oder Radiologen, erzeugt wurden,
- die Nummer eines Organprogramms,
- Alignmentdaten,
- eine Anweisung zur Wiederholung der Bildaufnahme,
- adjustierte Parameterdaten für eine wiederholte Bildaufnahme,
- Befundungsdaten, vorzugsweise radiologische Daten.

Weitere Datenquellen für Ausgangstrainingsdaten sind zum Beispiel aus den Bilddaten gewonnene strukturelle Parameter zur Bildqualität, wie zum Beispiel das Signal/Rauschverhältnis oder die Artefaktstärke von Bewegungsartefakten.

Die genannten Ausgangstrainingsdaten bilden das jeweilige Ergebnis eines jeweiligen Teilprozesses eines Bildgebungsprozesses oder Befundungsprozesses. Ein solcher Teilprozess kann die Anpassung des bildgebenden Systems an eine Indikation, die Bildgebungsplanung auf Basis einer Scout-Bildgebung, die eigentliche Bildgebung, den Befundungsprozess sowie eine Entscheidung, ob eine Bildgebung mit einem Kontrastmittel im Rahmen eines sogenannten Refinement-Scans wiederholt werden soll, betreffen.

In einer besonders effektiven Ausgestaltung des erfindungsgemäßen Befundungsverfahrens ist das automatisierte Steuerungssystem gemäß dem erfindungsgemäßen Verfahren zum Bereitstellen eines trainierten automatisierten Steuerungssystems trainiert. Vorteilhaft kann das beschriebene Trainingsverfahren auch für eine Automatisierung der Befundung genutzt werden. Durch eine solche Automatisierung lässt sich Personal einsparen und die Verfügbarkeit und Zuverlässigkeit der Befundung erhöhen.

Die Erfindung betrifft auch ein Trainingssystem, das eine erste Trainingsschnittstelle zum Empfangen von Eingangstrainingsdaten, eine zweite Trainingsschnittstelle zum Empfangen von Ausgangstrainingsdaten, wobei die Ausgangstrainingsdaten mit den Eingangstrainingsdaten (TE) zusammenhängen, eine Trainingsrechnereinheit zum Trainieren einer Funktion zur automatisierten Steuerung basierend auf den Eingangstrainingsdaten und den Ausgangstrainingsdaten, und eine dritte Trainingsschnittstelle zum Bereitstellen der Trainingsfunktion für ein automatisiertes Steuerungssystem aufweist.

Weiterhin betrifft die Erfindung ein Computerprogrammprodukt mit einem Computerprogramm, welches direkt in eine cloudbasierte Speichereinrichtung ladbar ist, mit Programmabschnitten, um alle Schritte eines erfindungsgemäßen Verfahrens auszuführen, wenn das Computerprogramm cloudbasiert ausgeführt wird. Darüber hinaus betrifft die Erfindung ein computerlesbares Medium, auf welchem von einer Rechnereinheit einlesbare und ausführbare Programmabschnitte gespeichert sind, um alle Schritte eines erfindungsgemäßen Verfahrens auszuführen, wenn die Programmabschnitte von der Rechnereinheit ausgeführt werden.

Das Trainingssystem, das Computerprogrammprodukt und das computerlesbare Medium teilen die Vorteile der erfindungsgemäßen Verfahren.

Die Erfindung wird im Folgenden unter Hinweis auf die beigefügten Figuren anhand von Ausführungsbeispielen noch einmal näher erläutert. Es zeigen:

FIG 1 ein Flussdiagramm, welches ein dezentralisiert gesteuertes Bildgebungsverfahren gemäß einem ersten Ausführungsbeispiel der Erfindung veranschaulicht,

FIG 2 eine schematische Darstellung eines dezentralisierten bildgebungsbasierten Datengewinnungssystems gemäß einem Ausführungsbeispiel der Erfindung,

FIG 3 ein Flussdiagramm, welches ein automatisiertes dezentralisiert gesteuertes Bildgebungsverfahren gemäß einem zweiten Ausführungsbeispiel der Erfindung veranschaulicht,

FIG 4 ein Flussdiagramm, welches ein dezentralisiert gesteuertes Bildgebungsverfahren gemäß einem Ausführungsbeispiel der Erfindung im Detail veranschaulicht,

FIG 5 ein Flussdiagramm, welches ein vollständig automatisiertes dezentralisiert gesteuertes Bildgebungsverfahren gemäß einem Ausführungsbeispiel der Erfindung veranschaulicht,

FIG 6 eine schematische Darstellung eines künstlichen neuronalen Netzes,

FIG 7 ein Flussdiagramm, welches ein Trainingsverfahren zum Trainieren einer Vorbereitung einer Bildgebung auf Basis einer Indikationswahl veranschaulicht,

FIG 8 ein Flussdiagramm, welches ein Trainingsverfahren zum Trainieren einer Wahl eines Field of View und einer Abbildungsschicht veranschaulicht,

FIG 9 ein Flussdiagramm, welches ein Trainingsverfahren zum Trainieren einer Qualtitätskontrolle von Bilddaten und einer Entscheidung, ob eine Bildaufnahme mit entsprechend geänderten Parametern wiederholt werden soll, veranschaulicht,

FIG 10 ein Flussdiagramm, welches ein Trainingsverfahren zum Trainieren eines Befundungsvorgangs auf Basis von Bilddaten veranschaulicht,

FIG 11 ein Flussdiagramm, welches ein Trainingsverfahren zum Trainieren eines Prüfungsvorgangs, ob eine Befundung ausreichend zuverlässig ist oder ob eine Bildgebung mit Kontrastmittel durchgeführt werden soll, veranschaulicht,

FIG 12 eine schematische Darstellung eines Trainingssystems gemäß einem Ausführungsbeispiel der Erfindung,

FIG 13 eine schematische Darstellung eines cloudbasierten dezentralisierten Bilddatengewinnungssystems und Trainingsdatengewinnungssystems gemäß einem Ausführungsbeispiel der Erfindung.

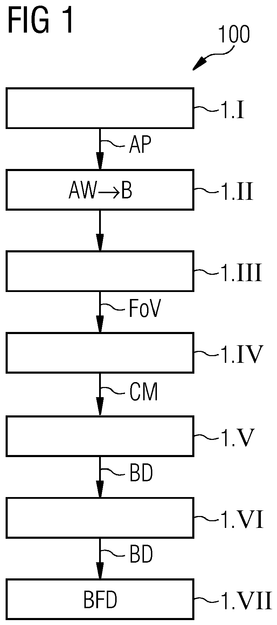

In FIG 1 ist ein Flussdiagramm 100 gezeigt, welches ein automatisiertes bildgebungsbasiertes Datengewinnungsverfahren gemäß einem ersten Ausführungsbeispiel der Erfindung veranschaulicht. Bei dem ersten Ausführungsbeispiel wird ein fernmündlich gesteuertes Bildgebungsverfahren realisiert. Im Vorfeld des in FIG 1 veranschaulichten Verfahrens begibt sich ein Benutzer B, zum Beispiel ein Krankenpfleger gemeinsam mit dem Patienten zu einer medizintechnischen Bildgebungsanlage, in diesem konkreten Ausführungsbeispiel eine MR-Anlage, und schaltet das System ein. Bei dem Schritt 1.I drückt der Benutzer B einen Knopf, der direkt an der Scaneinheit der MR-Anlage angeordnet ist. Dadurch wird ein cloudbasiertes Anwendungsprogramm AP ausgelöst, welches nun selbstständig eine über die Cloud bzw. ein Datennetz verfügbare medizinischtechnische Assistentin MTA sucht und eine Sprach- und Bildverbindung zwischen der Assistentin MTA und dem Benutzer B herstellt. Die Assistentin MTA hat nun die Benutzerschnittstelle sowie den gesamten Untersuchungsraum über eine IP-Kamera im Blick. Bei dem Schritt 1.II erhält der Benutzer B nun über die Sprachverbindung Anweisungen AW für die Patientenregistrierung. Anschließend wird bei dem Schritt 1.III die Auswahl der jeweiligen Untersuchungsregion FOV von der Assistentin MTA betreut. Bei dem Schritt 1.IV erfolgt ein Sprachdialog über weitere Protokollparameter, wie zum Beispiel den Einsatz eines Kontrastmittels CM und ähnliches. Dabei gibt der Benutzer B im Dialog mit dem Patienten P und der Assistentin MTA entsprechende Protokolldaten über die Benutzerschnittstelle ein. Anschließend wird bei dem Schritt 1.V die Bildgebung von dem Untersuchungsbereich durchgeführt, wobei auch dieser Vorgang aus der Ferne von der Assistentin MTA, beispielsweise mit Hilfe einer netzbasierten Kamera überwacht wird. Nach Abschluss der Bildgebung wird bei dem Schritt 1.VI von einer cloudbasierten Netzapplikation ein möglicherweise an einem weit entfernten Ort befindlicher verfügbarer Radiologe gesucht und die Bilddaten BD werden über das Internet an den verfügbaren Radiologen R übermittelt, so dass ohne Wartezeit eine Befundung anhand der erzeugten Bilddaten BD erfolgen kann. Bei dem Schritt 1.VII werden die Befundungsdaten BFD über das Internet an den Benutzer B und den Patienten P übermittelt und gleichzeitig in der Cloud gespeichert.

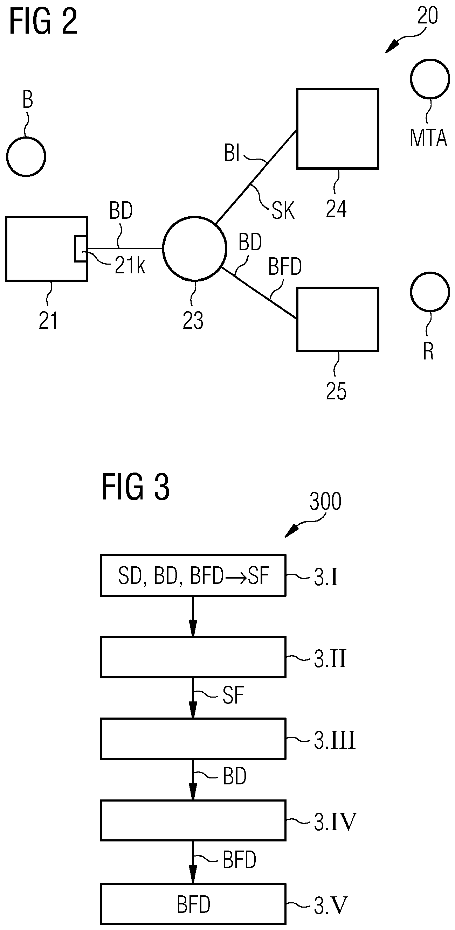

In FIG 2 ist eine schematische Darstellung eines cloudbasierten dezentralisierten bildgebungsbasierten Datengewinnungssystems 20 gemäß einem ersten Ausführungsbeispiel der Erfindung gezeigt. Das Datengewinnungssystem 20 umfasst eine MR-Bildgebungseinrichtung 21, in der ein Patient (nicht gezeigt) für eine bildgebende Untersuchung platzierbar ist. Die MR-Bildgebungseinrichtung 21 wird von einem Benutzer B bedient, welcher in FIG 2 als Kreis symbolisiert ist. Der Benutzer B verbindet sich unter Anwendung einer der MR-Bildgebungseinrichtung 21 zugeordneten Kommunikationseinrichtung 21k mit Hilfe einer in einem Datennetz 23, das als Cloud ausgebildet sein kann, gespeicherten Netzapplikation mit einem Expertenarbeitsplatz 24, über den eine medizinischtechnische Assistentin MTA, welche in FIG 2 auch als Kreis symbolisiert ist, eine sprachliche Kommunikation SK führen kann und Bildinformationen BI von der Situation der Bildgebung vor Ort erhalten kann. Die Bildinformationen BI, welche das Szenario vor Ort, d.h. am Ort der MR-Bildgebungseinrichtung 21 zeigen, können zum Beispiel von einer Kamera, welche sich in dem Raum, in dem die MR-Bildgebungseinrichtung angeordnet ist, befindet, aufgenommen werden. Teil des Systems 20 ist auch ein Expertenarbeitsplatz 25, welchem die von der MR-Bildgebungseinrichtung 21 erzeugten Bilddaten BD über das Datennetz 23 übermittelbar sind. Die von einem Radiologen R, welcher in FIG 2 auch durch einen Kreis symbolisiert ist, auf Basis dieser Bilddaten BD an dem Expertenarbeitsplatz 25 erzeugten Befundungsdaten BFD lassen sich über das Netz 23 an den Benutzer B übermitteln. Weiterhin können die bei der Bildgebung und bei der Befundung erfassten und erzeugten Daten BD, BFD in der Cloud 23 gespeichert werden, um für spätere Untersuchungen, die eventuell an einem anderen Ort stattfinden, nutzbar zu sein.

In FIG 3 ist ein Flussdiagramm 300 gezeigt, welches ein automatisiertes bildgebungsbasiertes Datengewinnungsverfahren gemäß einem zweiten Ausführungsbeispiel der Erfindung veranschaulicht. Anders als bei dem ersten Ausführungsbeispiel erfolgt bei dem zweiten Ausführungsbeispiel die Steuerung der Bildgebung sowie des Befundungsprozesses mit Hilfe von künstlicher Intelligenz. Hierzu wird zunächst bei dem Schritt 3.I auf Basis der bei dem in FIG 1 veranschaulichten Verfahren erzeugten Steuerdaten SD, Bilddaten BD und Befundungsdaten BFD eine auf einem neuronalen Netz SF basierende automatisierte cloudbasierte Steuerung des Bildgebungsprozesses und eine automatisierte Befundung trainiert. Das trainierte neuronale Netz SF wird nun bei dem Schritt 3.II als Applikation für einen Benutzer B über das Internet zur Verfügung gestellt. Beispielsweise nimmt eine Scan-Applikation die Aufgabe einer medizinisch-technischen Assistentin MTA wahr. D.h., der Benutzer B führt nun bei dem Schritt 3.III zur Vorbereitung und Durchführung eines Bildgebungsvorgangs einen Dialog mit der Scan-Applikation, ohne dass eine entsprechend qualifizierte Person an dem Vorgang beteiligt ist. Anschließend erfolgt bei dem Schritt 3.IV ebenfalls über eine NetzApplikation eine automatisierte Befundung, an der wiederum keine entsprechend qualifizierte Person beteiligt sein muss. Bei dem Schritt 3.V ruft der Benutzer B die erzeugten Befundungsdaten BFD ab und beendet damit den Untersuchungsprozess.

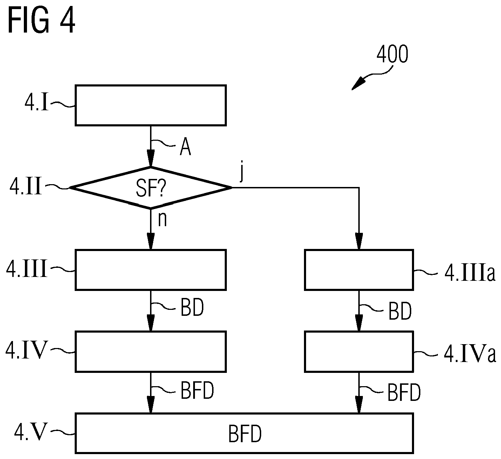

In FIG 4 ist ein Flussdiagramm 400 veranschaulicht, welches ein automatisiertes bildgebungsbasiertes Datengewinnungsverfahren gemäß einem Ausführungsbeispiel der Erfindung zeigt. Bei dem in FIG 4 veranschaulichten Ausführungsbeispiel wird ein vollautomatisierter Untersuchungsprozess realisiert. Bereits die Anforderung A einer Bildgebung und/oder einer Befundung wird nun bei dem Schritt 4.I direkt an ein im Datenübertragungsnetz gespeichertes Programm, welches auf einem künstlichen neuronalen Netzwerk basiert, ohne menschlichen Assistenten übermittelt. Dieses Programm klassifiziert nun bei dem Schritt 4.II die gewünschten Prozesse danach, ob sie virtuell oder automatisiert bearbeitet werden können. Die Klassifizierung hängt insbesondere davon ab, ob für eine automatisierte Anwendung ein Anwendungsprogramm, welches auf einem trainierten künstlichen neuronalen Netz SF basiert, zur Verfügung steht oder nicht. Anschließend erfolgt die Steuerung der Bildgebung und anschließend des Befundungsprozesses in Abhängigkeit von der bei dem Schritt 4.II durchgeführten Klassifikation. Die Steuerung kann, wie bei dem Schritt 4.III und 4.IV realisiert, virtuell, d.h. unter Einsatz einer fernsteuernden qualifizierten Bedienperson ausgeführt werden oder alternativ bei dem Schritt 4.IIIa und bei dem Schritt 4.IVa kann die Steuerung der Bildgebung und der Befundungsprozesses automatisiert ausgeführt werden. Bei dem Schritt 4.V werden schließlich die Befundungsdaten BFD an den Benutzer B übermittelt.

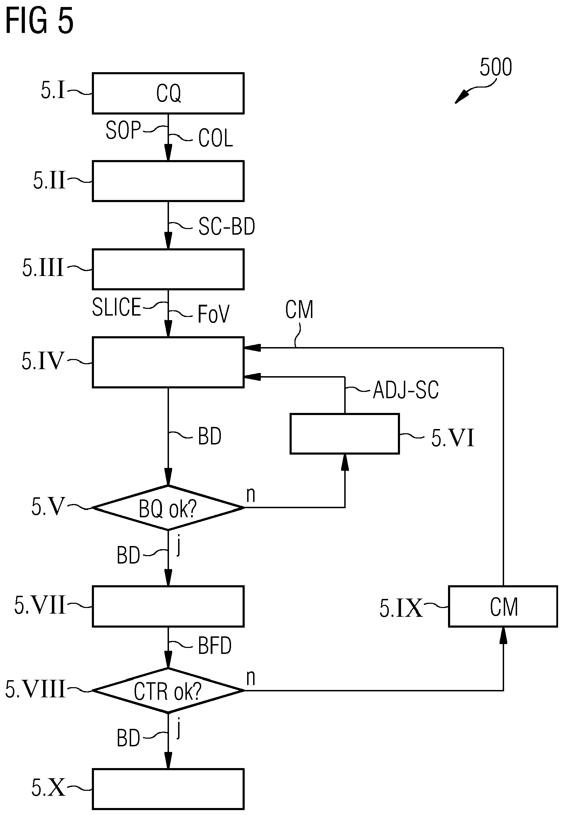

In FIG 5 wird ein Flussdiagramm 500 gezeigt, welches ein vollständig automatisiertes dezentralisiert gesteuertes Bildgebungsverfahren gemäß einem Ausführungsbeispiel der Erfindung veranschaulicht. Das in FIG 5 veranschaulichte Ausführungsbeispiel soll im Detail veranschaulichen, was unter Steuerung der Bildgebung sowie der Befundung im Einzelnen zu verstehen ist.

Bei dem Schritt 5.I wird zunächst auf Basis einer klinischen Fragestellung CQ, die mit Hilfe einer Bildgebung beantwortet werden soll, eine Voreinstellung für eine medizintechnische Bildgebung ermittelt. Beispielsweise soll geklärt werden, ob ein Organ eines Patienten von einem Tumor befallen ist oder nicht. Die Voreinstellung umfasst bei einem MR-System als medizintechnisches bildgebendes System beispielsweise einen geeigneten Satz SOP von Bildgebungsprotollen und eine Auswahl COL von für die Abbildung geeigneten Spulen.

Bei dem Schritt 5.II wird ein Scoutbild SC-BD von dem Patienten erzeugt, um die Lage des Patienten und seiner Organe zu ermitteln. Dieses Scoutbild SC-BD wird bei dem Schritt 5.III dazu genutzt den Untersuchungsbereich FoV, auch als Field of View bezeichnet, für die nachfolgende MR-Bildgebung sowie abzubildende Schichten SLICE festzulegen.

Bei dem Schritt 5.IV erfolgt der eigentliche Bildgebungsvorgang von dem festgelegten Untersuchungsbereich FoV. Die dabei erzeugten Bilddaten BD werden dann bei dem Schritt 5.V auf hinreichende Qualität BQ geprüft. Ist die Bildqualität nicht ausreichend, was in FIG 5 mit "n" gekennzeichnet ist, so wird zu dem Schritt 5.VI übergegangen. Bei dem Schritt 5.VI wird eine Parameteradjustierung ADJ-SC vorgenommen und anschließend wird zu dem Schritt 5.IV zurückgekehrt, bei dem eine erneute MR-Bildaufnahme mit adjustierten Parametern erfolgt. Wird bei dem Schritt 5.V dagegen ermittelt, dass die Bildqualität BQ der ersten Bildaufnahme ausreichend ist, was in FIG 5 mit "j" gekennzeichnet ist, so wird zu dem Schritt 5.VII übergegangen, bei dem die qualitätsgeprüften Bilddaten BD einer radiologischen Befundung unterzogen werden. Die dabei erzeugten Befundungsdaten BFD werden anschließend bei dem Schritt 5.VIII auf ihre Zuverlässigkeit CTR hin geprüft. Wird bei dem Schritt 5.VIII ermittelt, dass die Befundung nicht zuverlässig ist, was in FIG 5 mit "n" gekennzeichnet ist, so wird zu dem Schritt 5.IX übergegangen, bei dem ein Kontrastmittel CM und dessen Dosierung für eine erneute Bildgebung gewählt wird. Anschließend wird zu dem Schritt 5.IV zurückgekehrt und mit dem Kontrastmittel CM die Bildgebung wiederholt usw.. Wird dagegen bei dem Schritt 5.VIII ermittelt, dass das Befundungsergebnis BFD hinreichend zuverlässig ist, was in FIG 5 mit "n" gekennzeichnet ist, so wird zu dem Schritt 5.X übergegangen, bei dem das Befundungsergebnis BFD an den Benutzer B übermittelt wird.

Die in FIG 5 veranschaulichten Schrittegruppen 5.I, 5.II bis 5.III, 5.IV bis 5.VI, 5.VII und 5.VIII bis 5.X können jeweils durch Anwendung eines cloudbasierten Computerprogramms, dessen Algorithmus durch Anwendung eines künstlichen neuronalen Netzes erzeugt wurde, automatisiert durchgeführt werden.

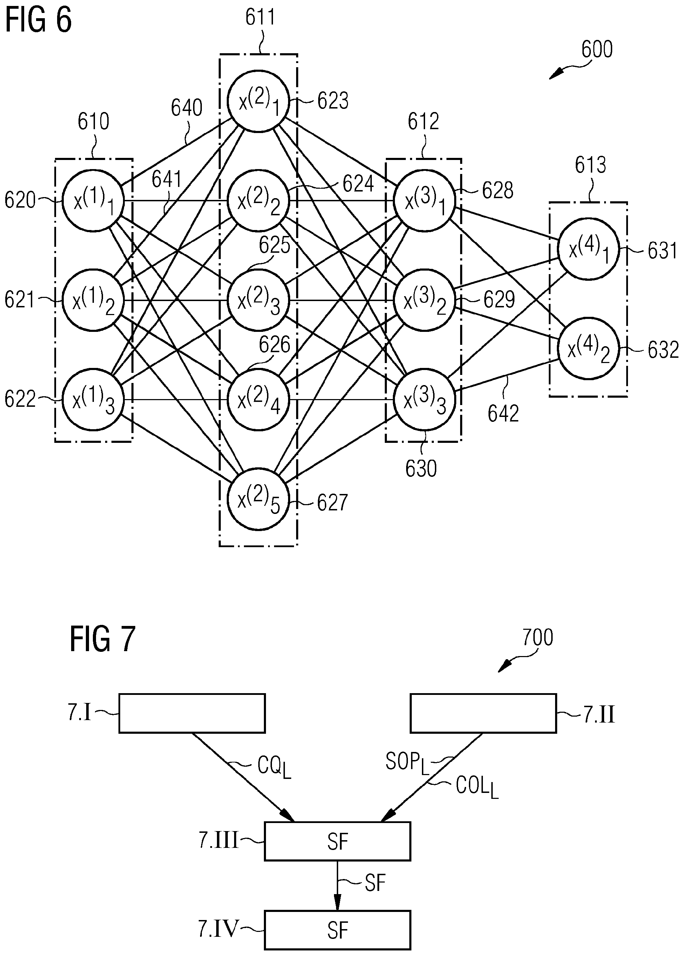

Ein solches künstliches neuronales Netz 600 ist in FIG 6 schematisch dargestellt. Alternative Bezeichnungen sind "neuronales Netzwerk", "künstliches neuronales Netz" oder "neuronales Netz".

Das künstliche neuronale Netzwerk 600 weist Knoten 620, ..., 632 und Kanten 640,..., 642 auf, wobei jede Kante 640, ..., 642 eine gerichtete Verbindung von einem ersten Knoten 620, ..., 632 zu einem zweiten Knoten 620, ..., 632 ist. Im Allgemeinen sind der erste Knoten 620, ..., 632 und der zweite Knoten 620, ..., 632 unterschiedliche Knoten 620, ..., 632. Es ist auch möglich, dass der erste Knoten 620, ..., 632 und der zweite Knoten 620, ..., 632 identisch sind. Beispielsweise ist in FIG 6 die Kante 640 eine gerichtete Verbindung von dem Knoten 620 zu dem Knoten 623 und die Kante 642 ist eine gerichtete Verbindung von dem Knoten 630 zu dem Knoten 632. Eine Kante 640, ..., 642 von einem ersten Knoten 620, ..., 632 zu einem zweiten Knoten 620,..., 632 wird auch als "eingehende Kante" für den zweiten Knoten 620,..., 632 und als "ausgehende Kante" für den ersten Knoten 620,..., 632 bezeichnet.

In diesem Ausführungsbeispiel können die Knoten 620, ..., 632 des künstlichen neuronalen Netzwerks 600 in Schichten 610, ..., 613 angeordnet sein, wobei die Schichten eine intrinsische Reihenfolge aufweisen können, die durch die Kanten 640,..., 642 zwischen den Knoten 620,..., 632 festgelegt wird. Insbesondere die Kanten 640, ..., 642 können nur zwischen benachbarten Schichten oder Knoten auftreten. In dem veranschaulichten Ausführungsbeispiel gibt es eine Eingangsschicht 610, welche nur Knoten 620,..., 622 ohne eingehende Kante aufweist, eine Ausgangsschicht 613, welche nur Knoten 631, 632 ohne ausgehende Kanten aufweist, und versteckte Schichten 611, 612 zwischen der Eingangsschicht 610 und der Ausgangsschicht 613. Im Allgemeinen kann die Anzahl der versteckten Schichten 611, 612 beliebig gewählt werden.

Die Anzahl der Knoten 620,..., 622 innerhalb der Eingangsschicht 610 ist üblicherweise der Anzahl von Eingangswerten des neuronalen Netzwerks zugeordnet und die Anzahl der Knoten 631, 632 innerhalb der Ausgangsschicht 613 ist der Anzahl der Ausgangswerte des neuronalen Netzwerks zugeordnet. Insbesondere kann eine reelle Zahl als Wert jedem Knoten 620, ..., 632 des neuronalen Netzwerks 600 zugeordnet werden. Hier bezeichnet $x^{(n)}_i$ den Wert des i-ten Knotens 620, ..., 632 der n-ten Schicht 610,..., 613. Die Werte der Knoten 620, ..., 622 der Eingangsschicht 610 entsprechen den Eingangswerten des neuronalen Netzwerks 600, die Werte der Knoten 631, 632 der Ausgangsschicht 613 entsprechen den Ausgangswerten des neuronalen Netzwerks 600. Weiterhin kann jede Kante 640,..., 642 ein Gewicht aufweisen, das eine reelle Zahl, ist, insbesondere, kann das Gewicht eine reelle Zahl in dem Intervall [-1, 1] oder innerhalb des Intervalls [0, 1] aufweisen. Hier bezeichnet $w^{(m,n)}_{i,j}$ das Gewicht der Kante zwischen dem i-ten Knoten 620,..., 632 der m-ten Schicht 610,..., 613 und dem j-ten Knoten 620,..., 632 der n-ten Schicht 610,..., 613. Weiterhin ist die Abkürzung $w^{(n)}_{i,j}$ für das Gewicht $w^{(n, n+1)}_{i,j}$ definiert.

Um die Ausgangswerte des neuronalen Netzwerks 100 zu berechnen, werden die Eingangswerte durch das neuronale Netzwerk propagiert. Die Werte der Knoten 620, ..., 632 der (n+1)-ten Schicht 610, ..., 613 können basierend auf den Werten der Knoten 620,..., 632 der n-ten Schicht 510, ..., 613 durch

$$x^{(n+1)}_j = f\left(\sum_i x^{(n)}_i w^{(n)}_{i,j}\right)$$

berechnet werden.

Dabei ist die Funktion f eine Transferfunktion (eine andere Bezeichnung ist "Aktivierungsfunktion"). Bekannte Transferfunktionen sind Stufenfunktionen, Sigmoidfunktionen (zum Beispiel die logistische Funktion, die verallgemeinerte logistische Funktion, der Tangens Hyperbolicus, die Arcustangens-Funktion, die Fehlerfunktion, die Smoothstep-Funktion) oder Gleichrichter-Funktionen. Die Transferfunktion wird hauptsächlich für Normalisierungszwecke genutzt.

Die Werte $x^{(n)}_i$ werden schichtweise durch das neuronale Netzwerk propagiert, wobei die Werte der ersten versteckten Schicht 611 basierend auf den Werten der Eingangsschicht 610 des neuronalen Netzes berechnet werden können und wobei die Werte der zweiten versteckten Schicht 612 basierend auf den Werten der ersten versteckten Schicht 611 berechnet werden können.

Um die Werte $w^{(m,n)}_{i,j}$ für die Kanten festzulegen, muss das neuronale Netz 600 unter Verwendung von Trainingsdaten trainiert werden. Die Trainingsdaten weisen Trainingseingangsdaten und Trainingsausgangsdaten auf (bezeichnet mit $y_i$). Für einen Trainingsschritt wird das neuronale Netz 600 auf die Trainingseingangsdaten angewendet, um berechnete Ausgangsdaten zu erzeugen. Die Trainingsdaten und die berechneten Ausgangsdaten weisen eine Anzahl von Werten auf, deren Anzahl gleich der Anzahl der Knoten der Ausgangsschicht ist.

Ein Vergleich zwischen den berechneten Ausgangsdaten und den Trainingsdaten wird verwendet, um die Gewichte innerhalb des neuronalen Netzwerks 600 rekursiv anzupassen (hierzu wird ein Backpropagation-Algorithmus angewendet). Die Gewichte werden entsprechend der folgenden Formel geändert:

$$w'^{n}_{i,j} = w^{(n)}_{i,j} - \gamma \delta^{(n)}_j \cdot x^{(n)}_i,$$

wobei $\gamma$ eine Lernrate ist und die Zahlen $\delta^{(n)}_j$ rekursiv berechnet werden können zu:

$$\delta^{(n)}_j = \left( \sum_k \delta^{(n+1)}_k \cdot w^{(n+1)}_{j,k} \right) \cdot f'\left( \sum_i x^{(n)}_{i,j} \cdot w^{(n)}_{i,j} \right)$$

Basierend auf $\delta^{(n+1)}_j$, wenn die (n+1)-te Schicht nicht die Ausgangsschicht ist, und

$$\delta^{(n)}_j = \left( x^{(n+1)}_k - y^{(n+1)}_j \right) \cdot f'\left( \sum_i x^{(n)}_{i,j} \cdot w^{(n)}_{i,j} \right),$$

wenn die (n+1)-te Schicht die Ausgangsschicht 613 ist, wobei f' die erste Ableitung der Aktivierungsfunktion ist und $y^{(n+1)}_j$ der Vergleichstrainingswert für den j-ten Knoten der Ausgangsschicht 613 ist.

In FIG 7 ist ein Flussdiagramm 700 gezeigt, welches ein Trainingsverfahren zum Trainieren einer Vorbereitung einer Bildgebung auf Basis einer Indikationswahl veranschaulicht. Bei dem Schritt 7.I werden gelabelte Fragestellungsdaten $CQ_L$ aus einer Trainingsdatenbank gelesen. Weiterhin werden bei dem Schritt 7.II Protokolldaten $SOP_L$ sowie Daten für Spulensätze $COL_L$ aus einer Trainingsdatenbank gelesen. Die Trainingsdaten werden anschließend bei dem Schritt 7.III zum Training eines neuronalen Netzes SF genutzt. Schließlich wird das trainierte neuronale Netz SF bei dem Schritt 7.IV für eine automatisierte Bildgebungsvorbereitung zur Verfügung gestellt.

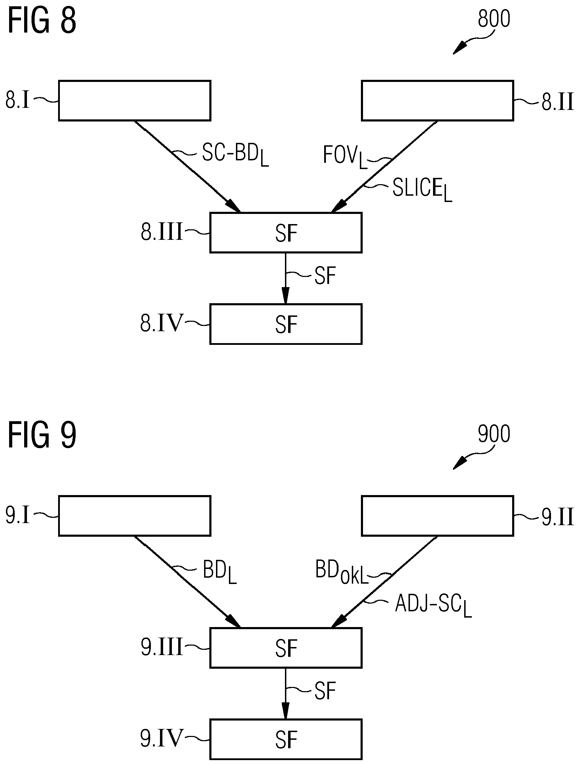

In FIG 8 ist ein Flussdiagramm 800, welches ein Trainingsverfahren zum Trainieren eines sogenannten Auto-Align-Prozesses einer Bildgebung veranschaulicht, gezeigt. Bei dem Schritt 8.I werden gelabelte Scout-Bilddaten SD-$BD_L$ aus einer Trainingsdatenbank ausgelesen. Zusätzlich werden bei dem Schritt 8.II gelabelte Daten $FoV_L$ zur Festlegung des abzubildenden Bereichs sowie gelabelte Daten $SLICE_L$ zur Festlegung von abzutastenden Schichten bei der späteren Bildgebung aus der Trainingsdatenbank ausgelesen. Die Trainingsdaten SD-$BD_L$, $FoV_L$, $SLICE_L$ werden anschließend bei dem Schritt 8.III zum Training eines neuronalen Netzes SF genutzt. Schließlich wird das trainierte neuronale Netz SF bei dem Schritt 8.IV für einen automatisierten Auto-Align-Prozess zur Verfügung gestellt.

In FIG 9 ist ein Flussdiagramm 900, welches ein Trainingsverfahren zum Trainieren der Steuerung des eigentlichen Bildgebungsprozesses veranschaulicht, gezeigt. Bei dem Schritt 9.I werden gelabelte Bilddaten $BD_L$ aus einer Trainingsdatenbank ausgelesen. Zusätzlich werden bei dem Schritt 9.II gelabelte Daten $BD_{okL}$, die darüber Auskunft geben, ob die Qualität der gelabelten Bildddaten $BD_L$ ausreichend ist. Bei dem Schritt 9.II werden auch gelabelte Daten ADJ-$SC_L$ aus der Trainingsdatenbank ausgelesen, welche Auskunft über eine Anpassung von Parametern einer zu wiederholenden Bildgebung geben. Diese Daten werden benötigt, falls die Bildqualität der Bilddaten $BD_L$ der ersten Bildgebung als nicht ausreichend eingestuft sind. Die Trainingsdaten $BD_L$, $BD_{okL}$, ADJ-$SC_L$ werden anschließend bei dem Schritt 9.III zum Training eines neuronalen Netzes SF genutzt. Schließlich wird das trainierte neuronale Netz SF bei dem Schritt 9.IV für einen automatisierten Bildgebungsprozess zur Verfügung gestellt.

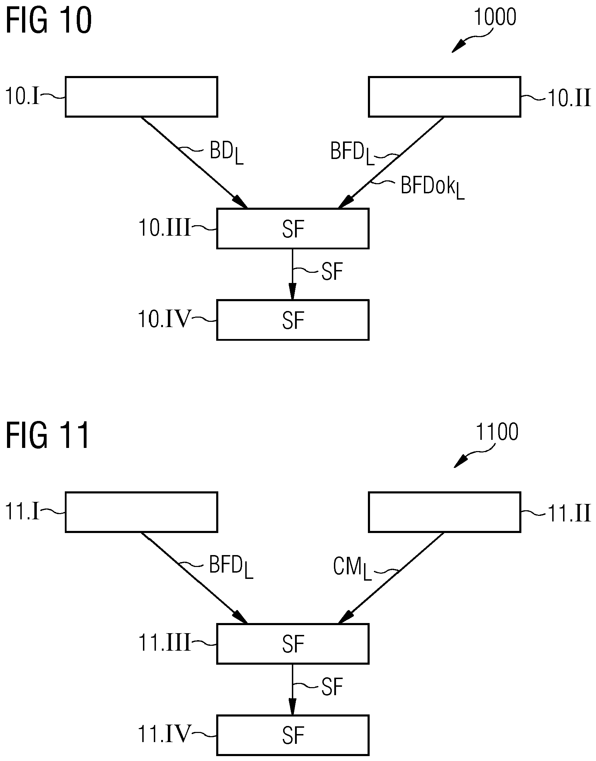

In FIG 10 ist ein Flussdiagramm 1000, welches ein Trainingsverfahren zum Trainieren eines automatisierten Befundungsvorgangs veranschaulicht, gezeigt. Bei dem Schritt 10.I werden gelabelte Bilddaten $BD_L$ aus einer Trainingsdatenbank ausgelesen. Zusätzlich werden bei dem Schritt 10.II gelabelte Befundungsdaten $BFD_L$ aus der Trainingsdatenbank ausgelesen. Weiterhin werden bei dem Schritt 10.II auch Informationen $BFD$-$ok_L$ darüber ausgelesen, ob das Befundungsergebnis ausreichend zuverlässig ist oder nicht. Die Trainingsdaten $BD_L$, BFDL, $BFDok_L$ werden anschließend bei dem Schritt 10.III zum Training eines neuronalen Netzes SF genutzt. Schließlich wird das trainierte neuronale Netz SF bei dem Schritt 10.IV für einen automatisierten Befundungsprozess zur Verfügung gestellt.

In FIG 11 ist ein Flussdiagramm 1100, welches ein Trainingsverfahren zum Trainieren eines automatisierten Refinement-Prozesses veranschaulicht, gezeigt. Bei dem Schritt 11.I werden gelabelte Befundungsdaten $BFD_L$ aus einer Trainingsdatenbank ausgelesen. Zusätzlich werden bei dem Schritt 11.II gelabelte Daten $CM_L$ aus der Trainingsdatenbank ausgelesen, welche Auskunft darüber geben, welches Kontrastmittel und mit welcher Dosierung für eine erneute verfeinerte Bildgebung eingesetzt werden soll.

Die Trainingsdaten $BFD_L$, $CM_L$ werden anschließend bei dem Schritt 11.III zum Training eines neuronalen Netzes SF genutzt. Schließlich wird das trainierte neuronale Netz SF bei dem Schritt 11.IV für einen automatisierten Befundungsprozess zur Verfügung gestellt.

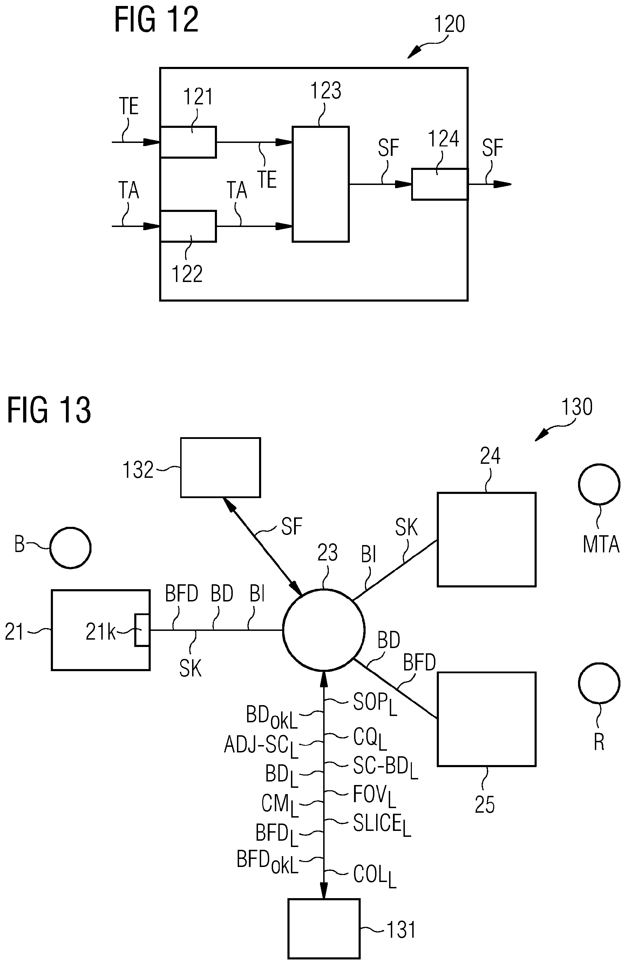

In FIG 12 ist eine schematische Darstellung eines Trainingssystems 120 gemäß einem Ausführungsbeispiel der Erfindung veranschaulicht. Das Trainingssystem 120 umfasst eine erste Trainingsschnittstelle 121 zum Empfangen von Eingangstrainingsdaten TE. Die Eingangstrainingsdaten können zum Beispiel die in FIG 7 bis FIG 11 beschriebenen gelabelten Fragestellungsdaten $CQ_L$, gelabelten Scout-Bilddaten $SD\text{-}BD_L$, gelabelten Bilddaten $BD_L$ oder gelabelten Befundungsdaten $BFD_L$ umfassen. Teil des Trainingssystems 120 ist auch eine zweite Trainingsschnittstelle 122 zum Empfangen von Ausgangstrainingsdaten TA. Die Ausgangstrainingsdaten TA können zum Beispiel die in FIG 7 bis FIG 11 beschriebenen gelabelten Protokolldaten $SOP_L$ sowie Daten für Spulensätze $COL_L$, gelabelte Daten $FOV_L$ zur Festlegung des abzubildenden Bereichs oder gelabelte Daten $SLICE_L$ zur Festlegung von abzutastenden Schichten bei der späteren Bildgebung, gelabelte Daten $BD_{okL}$, die darüber Auskunft geben, ob die Qualität der gelabelten Bildddaten $BD_L$ ausreichend ist, gelabelte Daten $ADJ\text{-}SC_L$, welche Auskunft über eine Anpassung von Parametern einer zu wiederholenden Bildgebung geben, oder Informationen $BFDok_L$ darüber, ob das Befundungsergebnis ausreichend zuverlässig ist oder nicht, und gelabelte Daten $CM_L$, welche Auskunft darüber geben, welches Kontrastmittel und mit welcher Dosierung für eine erneute verfeinerte Bildgebung eingesetzt werden soll, umfassen. Teil des Trainingssystems 120 ist auch eine Trainingsrechnereinheit 123 zum Trainieren einer Funktion SF zur automatisierten Bildgebungssteuerung basierend auf den Eingangstrainingsdaten TE und den Ausgangstrainingsdaten TA. Das Trainingssystem 120 weist auch eine dritte Trainingsschnittstelle 124 zum Bereitstellen der Trainingsfunktion SF für ein automatisiertes System auf.

In FIG 13 ist eine schematische Darstellung eines cloudbasierten dezentralisierten Bilddatengewinnungssystems 130 gemäß einem Ausführungsbeispiel der Erfindung gezeigt. Bei dem in FIG 13 gezeigten Ausführungsbeispiel sind zusätzlich zu den Zugängen 24, 25 für die medizinischtechnische Assistentin MTA und den Radiologen R auch noch auf neuronalen Netzen SF basierende künstliche Intelligenzen vorhanden, die alternativ zu den menschlichen Fachkräften die Bildgebung begleiten bzw. eine Befundung durchführen. Für das Training dieser softwarebasierten Dienste sind in der Cloud 23 entsprechende Trainingsprogramme vorhanden, die auf die in der Cloud gespeicherten Protokolldaten, Bilddaten und Befundungsdaten zugreifen. Das in FIG 13 beschriebene System 130 kann auch als Trainingsdatengewinnungssystem genutzt werden. Die manuell durch die medizinischtechnische Assistentin MTA sowie den Radiologen R erzeugten Trainingsdaten werden in einer Trainingsdatenbank 131 gespeichert. Zum Training einer Steuerungsfunktion bzw. eines künstlichen neuronalen Netzes SF können diese Daten aus der Trainingsdatenbank 131 ausgelesen werden. Die bei dem Trainingsprozess erzeugten künstlichen neuronalen Netze SF werden in einer Netzdatenbank 132 abgespeichert und können von der Cloud 23 jederzeit für eine Simulation einer medizinischtechnischen Assistentin MTA oder eines Radiologen R genutzt werden.

Auch für die Verwaltung des in FIG 13 gezeigten Systems kann ein neuronales Netz trainiert werden. Ein solches neuronales Netz kann eine Klassifikation von Aufträgen zur Bildgebung in für eine virtuelle qualifizierte Bedienperson MTA, R oder ein automatisiertes System SF geeignete Aufträge durchführen. Die als automatisiert bearbeitbar klassifizierten Aufträge können dann durch die in der Datenbank 132 bereits bereitstehenden neuronalen Netze, bzw. darauf basierende Computerprogramme bearbeitet werden. Die Klassifizierung kann zum Beispiel auf der Basis der Anzahl der für ein Training eines neuronalen Netzes vorhandenen Trainingsdaten erfolgen.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

**Patentansprüche**

1. Dezentralisiert gesteuertes Bildgebungsverfahren (100, 300, 400, 500), aufweisend die Schritte:

   - Ermitteln und Auswählen einer über ein Datenübertragungsnetz (23) mit einer Kommunikationseinrichtung (21k) einer medizintechnischen bildgebenden Anlage (21) verbindbaren Steuerungsinstanz (24),
   - Initialisieren einer netzbasierten Kommunikationsverbindung zwischen der Kommunikationseinrichtung (21k) der medizintechnischen bildgebenden Anlage (21) und der Steuerungsinstanz (24),
   - Fernsteuern eines Bildaufnahmevorgangs von einem Untersuchungsbereich (FoV) eines Patienten (P) durch die Steuerungsinstanz (24) über die netzbasierte Kommunikationsverbindung.

2. Verfahren nach Anspruch 1, wobei die Steuerungsinstanz (24) einen Expertenarbeitsplatz (24) aufweist, an welchem Steuerungsdaten erfasst und über die netzbasierte Kommunikationsverbindung zur Protokollerstellung und zur Durchführung des Bildgebungsverfahrens übermittelt werden.

3. Verfahren nach Anspruch 2, wobei das Fernsteuern des Bildaufnahmevorgangs auf Basis eines über die netzbasierte Kommunikationsverbindung geführten Dialogs zwischen einer qualifizierten Bedienperson (MTA) an dem Expertenarbeitsplatz (24) und einer am Ort der medizintechnischen bildgebenden Anlage (21) befindlichen Benutzerperson (B) erfolgt.

4. Befundungsdatengewinnungsverfahren, aufweisend die Schritte:

- Durchführen des Verfahrens nach einem der Ansprüche 1 bis 3,
- netzbasiertes Ermitteln und Auswählen eines mit der Kommunikationseinrichtung (21k) einer medizintechnischen bildgebenden Anlage (21) verbindbaren Expertenarbeitsplatzes (25) aus einer Mehrzahl von Expertenarbeitsplätzen, welcher optional einen Expertenarbeitsplatz für eine qualifizierte Bedienperson (R) aufweist,
- Übermitteln der bei dem Bildaufnahmevorgang erzeugten Bilddaten (BD) an den ausgewählten Expertenarbeitsplatz (25),
- Ausgeben von Befundungsdaten (BFD) von dem Expertenarbeitsplatz (25) .

5. Verfahren nach einem der Ansprüche 2 bis 4, wobei zumindest eine der qualifizierten Bedienpersonen (MTA, R) durch ein auf künstlicher Intelligenz basierendes, mit Hilfe von Trainingsdaten (TE, TA) trainiertes automatisiertes Steuerungssystem (SF, 23, 131, 132) simuliert wird.

6. Verfahren nach Anspruch 5, wobei ein Einsatz des trainierten automatisierten Steuerungssystems (SF, 23, 131, 132) in Abhängigkeit davon gestartet wird, ob eine ausreichende Anzahl von Trainingsdatensätzen (TE, TA) für den Trainingsprozess des jeweiligen automatisierten Steuerungssystems (SF, 23, 131, 132) zur Verfügung stehen.

7. Verfahren nach Anspruch 6, wobei eine Klassifikation von Aufträgen zur Bildgebung in Aufträge, welche für eine virtuelle qualifizierte Bedienperson (MTA, R) oder ein automatisiertes System (SF, 23) geeignet sind, auf künstlicher Intelligenz basierend durchgeführt wird.

8. Dezentralisiertes Bilddatengewinnungssystem (20, 130), aufweisend:

- eine medizintechnische bildgebende Anlage (21),
- eine Vermittlungseinrichtung (23) zum Ermitteln und Auswählen einer über ein Datenübertragungsnetz mit einer Kommunikationseinrichtung (21k) der medizintechnischen bildgebenden Anlage (21) verbindbaren Steuerungsinstanz (24),
- eine Initialisierungseinrichtung (23) zum Initialisieren einer netzbasierten Kommunikationsverbindung zwischen der Kommunikationseinrichtung (21k) der medizintechnischen bildgebenden Anlage (21) und der Steuerungsinstanz (24),
- die Steuerungsinstanz (24) zum Fernsteuern eines Bildaufnahmevorgangs von einem Untersuchungsbereich (FoV) eines Patienten (P) über die netzbasierte Kommunikationsverbindung.

9. System nach Anspruch 8, wobei die medizintechnische bildgebende Anlage (21) eine Anlage der folgenden Anlagentypen umfasst:

- eine CT-Anlage,
- eine MR-Anlage.

10. Befundungsdatengewinnungssystem, aufweisend:

- ein System (20, 130) nach Anspruch 8 oder 9,
- eine Vermittlungseinrichtung (23) zum netzbasierten Ermitteln und Auswählen eines mit der Kommunikationseinrichtung (21k) einer medizintechnischen bildgebenden Anlage (21) verbindbaren Expertenarbeitsplatzes (25) aus einer Mehrzahl von Expertenarbeitsplätzen, welcher optional einen Expertenarbeitsplatz für eine qualifizierte Bedienperson (R) aufweist,
- eine Initialisierungseinrichtung (23) zum Initialisieren einer netzbasierten Kommunikationsverbindung zwischen der Kommunikationseinrichtung (21k) der medizintechnischen bildgebenden Anlage (21) und dem ausgewählten Expertenarbeitsplatz (25) zum Übermitteln der bei dem Bildaufnahmevorgang erzeugten Bilddaten (BD) an den ausgewählten Expertenarbeitsplatz (25),
- eine Ausgangsschnittstelle zum Ausgeben von Befundungsdaten (BFD) von dem Expertenarbeitsplatz (25).

11. Verfahren zur Gewinnung von Trainingsdaten (TE, TA), vorzugsweise unter Anwendung eines Verfahrens nach einem der Ansprüche 1 bis 7, wobei

- ein automatisiertes Bilddatengewinnungssystem (130) mit einer medizintechnischen bildgebenden Anlage (21), einer Steuerungsinstanz (24), einer Kommunikationseinrichtung (21k) und einem automatisierten Steuerungssystem (SF, 23, 131, 132), vorzugsweise ein dezentralisiertes Bilddatengewinnungssystem (130) nach Anspruch 8 oder 9, eingerichtet wird,
- zur Gewinnung von Trainingsdaten (TE, TA, $CQ_L$, $COL_L$, $SOP_L$, $SLICE_L$, $FOC_L$, SC-$BD_L$, ADJ-$SC_L$, $BD_{oKL}$, $BD_L$, $BFD_L$, $CM_L$) das automatisierte Steuerungssystem (SF, 23, 131, 132) temporär durch einen Expertenarbeitsplatz (24, 25) mit einem Experten (MTA, R) ersetzt wird,
- eine Kommunikation zwischen dem Experten (MTA, R) und einer Benutzerperson (B) und/oder der bildgebenden medizintechnischen Anlage (21) protokolliert wird,
- optional die dabei protokollierten Daten (TE, TA, $CQ_L$, $COL_L$, $SOP_L$, $SLICE_L$, $FOC_L$, SC-$BD_L$, ADJ-$SC_L$, $BD_{oKL}$, $BD_L$, $BFD_L$, $CM_L$) aufbereitet werden
- und die protokollierten Daten (TE, TA, $CQ_L$, $COL_L$, $SOP_L$, $SLICE_L$, $FOC_L$, SC-$BD_L$, ADJ-$SC_L$, $BD_{oKL}$, $BD_L$, $BFD_L$, $CM_L$) und/oder aufbereiteten protokollierten Daten als Trainingsdaten (TE, TA) bereitgestellt werden.

12. Verfahren nach Anspruch 11, wobei die Trainingsdaten (TE, TA)

- als Eingangstrainingsdaten (TE) mindestens eine der folgenden Datenarten umfassen:

- Patientendaten,
- aufgabenspezifische Daten ($CQ_L$), vorzugsweise Untersuchungsdaten,
- die medizintechnische bildgebende Anlage (21) betreffende technische Daten,
- Scoutbilddaten (SC-$BD_L$),
- Bilddaten ($BD_L$)

- als Ausgangstrainingsdaten (TA) mindestens eine der folgenden Datenarten umfassen:

- Steuerungsdaten, die durch Experten (MTA, R) erzeugt wurden,
- die Nummer eines Organprogramms,
- Alignmentdaten ($SLICE_L$, $FOV_L$),
- eine Anweisung zur Wiederholung der Bildaufnahme,
- adjustierte Parameterdaten (ADJ-$SC_L$) für eine wiederholte Bildaufnahme,

- Befundungsdaten ($BFD_L$).

13. Verfahren zum Bereitstellen eines trainierten automatisierten Steuerungssystems, aufweisend die Schritte:

- Empfangen von gelabelten Eingangstrainingsdaten (TE),
- Empfangen von gelabelten Ausgangstrainingsdaten (TA), wobei die Ausgangstrainingsdaten (TA) mit den Eingangstrainingsdaten (TE) zusammenhängen,
- Trainieren einer Funktion (SF) basierend auf den Eingangstrainingsdaten (TE) und den Ausgangstrainingsdaten (TA),
- Bereitstellen der trainierten Funktion (SF) für ein automatisiertes Steuerungssystem (SF, 23, 131, 132).

14. Verfahren nach einem der Ansprüche 5 bis 7, wobei das automatisierte Steuerungssystem (SF, 23, 131, 132) gemäß dem Verfahren nach Anspruch 13 trainiert ist.

15. Trainingsdatengewinnungssystem, aufweisend:

- ein automatisiertes Bilddatengewinnungssystem mit

- einer medizintechnischen bildgebenden Anlage (21),
- einer Steuerungsinstanz (24),
- einer Kommunikationseinrichtung (21k) und
- einem automatisierten Steuerungssystem (SF, 23, 131, 132),

vorzugsweise ein dezentralisiertes Bilddatengewinnungssystem (130) nach Anspruch 8 oder 9,
- einen Expertenarbeitsplatz (24), welcher zur Gewinnung von Trainingsdaten (TA, TE) das automatisierte Steuerungssystem (SF, 23, 131, 132) temporär ersetzt,

- eine Protokolleinheit (23) zum Protokollieren der Kommunikation zwischen dem Experten (MTA) und einer Benutzerperson (B) und/oder der bildgebenden medizintechnischen Anlage (21) und
- optional eine Datenaufbereitungseinheit zum Aufbereiten der protokollierten Daten (TE, TA, $CQ_L$, $COL_L$, $SOP_L$, $SLICE_L$, $FOC_L$, $SC\text{-}BD_L$, $ADJ\text{-}SC_L$, $BD_{okL}$, $BD_L$, $BFD_L$, $CM_L$),
- eine Ausgangsschnittstelle zum Ausgeben der protokollierten Daten (TE, TA, $CQ_L$, $COL_L$, $SOP_L$, $SLICE_L$, $FOC_L$, $SC\text{-}BD_L$, $ADJ\text{-}SC_L$, $BD_{okL}$, $BD_L$, $BFD_L$, $CM_L$) und/oder aufbereiteten Daten als Trainingsdaten (TE, TA).

## FIG 1

100

| 1.I |
|---|

AP

| AW→B |
|---|

1.II

| |
|---|

1.III

FoV

| |
|---|

1.IV

CM

| |
|---|

1.V

BD

| |
|---|

1.VI

BD

| BFD |
|---|

1.VII

## FIG 2

## FIG 3

| | |
|---|---|
| SD, BD, BFD→SF | 3.I |
| | 3.II |
| | 3.III |
| | 3.IV |
| BFD | 3.V |

# FIG 4

400

| | |
|---|---|
| 4.I | |

↓ A

4.II — ⟨ SF? ⟩ —— j

n ↓

| | |
|---|---|
| 4.III | |

4.IIIa

↓ BD

↓ BD

| | |
|---|---|
| 4.IV | |

4.IVa

↓ BFD

↓ BFD

| | |
|---|---|
| 4.V | BFD |

FIG 5

# FIG 6

# FIG 7

## FIG 8

800

8.I

8.II

SC-BD$_L$

FOV$_L$

SLICE$_L$

8.III ⌐ SF

SF

8.IV ⌐ SF

## FIG 9

900

9.I

9.II

BD$_L$

BD$_{okL}$

ADJ-SC$_L$

9.III ⌐ SF

SF

9.IV ⌐ SF

## FIG 10

1000

10.I

10.II

$BD_L$

$BFD_L$

$BFDok_L$

10.III — SF

SF

10.IV — SF

## FIG 11

1100

11.I

11.II

$BFD_L$

$CM_L$

11.III — SF

SF

11.IV — SF

# FIG 12

120

# FIG 13

130

Europäisches Patentamt
European Patent Office
Office européen des brevets

**EUROPÄISCHER RECHERCHENBERICHT**

Nummer der Anmeldung

EP 19 17 4633

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2009/189988 A1 (JIA YUANYUAN [US] ET AL) 30. Juli 2009 (2009-07-30) * Zusammenfassung * * Absätze [0002], [0003], [0005], [0015], [0017], [0023]; Abbildung 1 * ----- | 1-15 | INV. G16H30/40 |
| A | EP 3 349 219 A1 (SIEMENS HEALTHCARE GMBH [DE]) 18. Juli 2018 (2018-07-18) * Zusammenfassung * * Absätze [0010] - [0011], [0038] - [0039], [0063]; Ansprüche 1,2,6 * ----- | 1-15 | |

RECHERCHIERTE SACHGEBIETE (IPC)

G16H

Der vorliegende Recherchenbericht wurde für alle Patentansprüche erstellt

| Recherchenort | Abschlußdatum der Recherche | Prüfer |
|---|---|---|
| München | 13. November 2019 | Schmitt, Constanze |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

..........................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 19 17 4633

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-11-2019

| Im Recherchenbericht angeführtes Patentdokument | Datum der Veröffentlichung | Mitglied(er) der Patentfamilie | Datum der Veröffentlichung |
|---|---|---|---|
| US 2009189988 A1 | 30-07-2009 | KEINE | |
| EP 3349219 A1 | 18-07-2018 | KEINE | |

EPO FORM P0461

Für nähere Einzelheiten zu diesem Anhang : siehe Amtsblatt des Europäischen Patentamts, Nr.12/82